# EUROPEAN PATENT APPLICATION

(11) **EP 4 725 395 A1**
(43) Date of publication of application: **15.04.2026**
(21) Application number: 24205309.8
(22) Date of filing: 08.10.2024
(51) Int. Cl.: A61B 5/00, A61B 5/103, A61B 5/107, A61B 5/11, A61B 8/00

(54) **SUBJECT POSITION DETERMINATION USING AN ARRAY OF SPATIALLY DISTRIBUTED PRESSURE SENSORS**

(71) Applicant: Koninklijke Philips N.V., 5656 AG Eindhoven (NL)
(72) Inventor: KRUEGER, Sascha, Eindhoven (NL); SENEGAS, Julien Thomas, Eindhoven (NL); WIEMKER, Rafael, 5656AG Eindhoven (NL); WIRTZ, Daniel, Eindhoven (NL); VAN EE, Raymond, Eindhoven (NL)
(74) Representative: Philips Intellectual Property & Standards

(57) **Abstract**

Disclosed herein is a medical system (100, 200, 400, 600) comprising a subject support (104) with an array of spatially distributed pressure sensors (108). The medical system further comprises a remote spatial mapping system (106, 106') configured for measuring a spatial map (132).

The execution of machine executable instructions (130) causes a computational system (120) to: repeatedly (306) acquire the spatial map from the remote spatial mapping system; repeatedly (308) acquire the array of pressure data; generate (302, 310) a subject position model (138) using the repeatedly acquired spatial map and the array of pressure data; repeatedly (312) acquire the array of pressure data (140) from the array of spatially distributed pressure sensors; repeatedly (314) receive the subject position by inputting the array of pressure data into the subject position model; and repeatedly (316) provide the subject position.

## Description

### FIELD OF THE INVENTION

The invention relates to medical imaging, in particular it relates to the detection and/or monitoring of subject motion during medical imaging procedures.

### BACKGROUND OF THE INVENTION

In various medical imaging modalities such as magnetic resonance imaging or computed tomography the subject may be inserted into a scanner. Cameras may be used to monitor various types of voluntary and involuntary subject motion. However, cameras are often times not able to image the complete subject during the imaging procedure.

United States patent application US2020121954A1 discloses an array of force sensors for determining a position of an object, detecting motion of object, and tracking motion of objects in 3D space are described herein. An array of force sensors can be used to monitor anatomical motion during medical procedures, such as head motion during cranial radiosurgery, to maintain a desired alignment with the anatomical feature. Alerts can be posted to the medical machine operator and the radiosurgery system or scanner can make compensatory adjustments to maintain the desired alignment either after suspension of treatment or dynamically during treatment.

### SUMMARY OF THE INVENTION

The invention provides for a medical system, a method of operating a medical system, and a computer program in the independent claims. Embodiments are given in the dependent claims.

In one aspect a medical system is disclosed. The medical system comprises a subject support comprising a subject surface configured for receiving a subject. The subject surface comprises an array of spatially distributed pressure sensors configured for measuring an array of pressure data. The medical system further comprises a remote spatial mapping system configured for measuring a spatial map that is descriptive of objects on the subject surface. The objects on the subject surface comprise the subject. The remote spatial mapping system is configured to register the spatial map to the array of spatially distributed pressure sensors. The medical system further comprises a memory storing machine-executable instructions and a computational system.

Execution of the machine-executable instructions causes the computational system to perform a calibration that comprises repeatedly acquiring the spatial map from the remote spatial mapping system and repeatedly acquiring the array of pressure data. The array of pressure data is temporally correlated to the spatial map. The execution of the machine-executable instructions further causes the computational system to generate a subject position model using the repeatedly acquired spatial map and the array of pressure data. The subject position model is configured to output a subject position in response to receiving the array of pressure data.

Execution of the machine-executable instructions further causes the computational system to perform subject motion tracking which comprises repeatedly acquiring the array of pressure data from the array of spatially distributed pressure sensors, repeatedly receiving the subject position by inputting the array of pressure data into the subject position model, and then repeatedly providing the subject position.

In another aspect a method of operating the medical system is disclosed. The medical system comprises a subject support comprising a subject surface configured for receiving a subject. The subject surface comprises an array of spatially distributed pressure sensors configured for measuring an array of pressure data. The medical system further comprises a remote spatial mapping system configured for measuring a spatial map that is descriptive of objects on the subject surface. The remote spatial mapping system is configured to register the spatial map to the array of spatially distributed pressure sensors. The method comprises performing a calibration. The calibration comprises repeatedly acquiring the spatial map from the remote spatial mapping system and repeatedly acquiring the array of pressure data. The array of pressure data is temporally correlated to the spatial map. The method further comprises generating a subject position model using the repeatedly acquired spatial map and the array of pressure data. The subject position model is configured to output a subject position in response to receiving the array of pressure data.

The method further comprises performing subject motion tracking. The subject motion tracking comprises repeatedly acquiring the array of pressure data from the array of spatially distributed pressure sensors, repeatedly receiving the subject position by inputting the array of pressure data into the subject position model, and repeatedly providing the subject position.

In another aspect, a computer program is disclosed. The computer program comprises machine-executable instructions. The execution of the machine-executable instructions causes a computational system to perform an example of a method of operating the medical system.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 illustrates a further example of a medical system.
Fig. 3 shows a flow chart which illustrates a method of using the medical system of Fig. 1 or of Fig. 2.
Fig. 4 illustrates a further example of a medical system.
Fig. 5 shows a flow chart which illustrates a method of using the medical system of Fig. 4.
Fig. 6 illustrates a further example of a medical system.
Fig. 7 illustrates an example of a subject support with an array of spatially distributed pressure sensors.
Fig. 8 shows a further view of the subject support of Fig. 7.
Fig. 9 illustrates the use of a mass model of a subject for calibrating a subject position model.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In an example, a medical system comprises a subject support comprising a subject surface configured for receiving a subject. The subject surface comprises an array of spatially distributed pressure sensors configured for measuring an array of pressure data.

The medical system further comprises a remote spatial mapping system configured for measuring a spatial map descriptive of objects on the subject surface. The objects on the subject surface comprise the subject. The remote spatial mapping system is configured to register the spatial map to the array of spatially distributed pressure sensors. As will be described below, the remote spatial mapping system may be implemented in different ways such as using imaging systems such as cameras, infra-red cameras, three-dimensional cameras as well as other systems such as ultrasound detection, LIDAR, and radar. The spatial map provides information on the distribution of objects on the subject surface.

The medical system further comprises a memory storing machine-executable instructions. The medical system further comprises a computational system. The execution of the machine-executable instructions causes the computational system to perform a calibration. The calibration comprises repeatedly acquiring the spatial map from the remote spatial mapping system and also repeatedly acquiring the array of pressure data. The array of pressure data is temporally correlated to the spatial map. For example, the array of pressure data and the spatial map may be timestamped to indicate the temporal correlation. In other examples, the spatial map and the array of pressure data acquired within a certain period of time are stored together as a single data structure.

Execution of the machine-executable instructions further causes the computational system to generate a subject position model using the repeatedly acquired spatial map and the array of pressure data. The subject position model is configured to output a subject position in response to receiving the array of pressure data. This generation of the subject position model may take different forms in different examples. In one example, the temporally correlated samples of the spatial map and the array of pressure data are used to generate the entire subject position model, for example a lookup table or a direct model. In other examples, there may be a pre-existing subject position model and then the generation of the subject position model is essentially a calibration or fitting or modification of the existing subject position model. For example, a neural network could be trained using the spatial maps and array of pressure data for a variety of individuals. This neural network may then be trained, for example, by modifying the output layer using the data acquired during the calibration.

Execution of the machine-executable instructions further causes the computational system to perform subject motion tracking. The subject motion tracking comprises repeatedly acquiring the array of pressure data from the array of spatially distributed pressure sensors and then repeatedly receiving the subject position by inputting the array of pressure data into the subject position model. Performing the motion tracking further comprises repeatedly providing the subject position.

This example may be beneficial because it may provide for an accurate means of monitoring the subject position without directly observing with the remote spatial mapping system. For example, before a subject is injected into the bore of a computed tomography or magnetic resonance imaging system they may lay on the subject support in full view of the remote spatial mapping system, which in this example may be a camera or three-dimensional camera system. As the subject sits there they go through a range of motions and then the array of pressure data for the different subject positions is able to accurately generate the subject position model. This for example may provide for improved medical imaging. Remote spatial mapping systems such as cameras are very good at monitoring small and also large movements of a subject. However, inside of a medical imaging system it can be very difficult if not impossible to take accurate measurements of the subject motion. Examples provide a means of correlating the measurements from an array of spatially distributed pressure sensors to a remote spatial mapping system. This may provide for accurate tracking of subject motion even when the subject is not visible to the remote spatial mapping system.

In patent application US2020121954A1, a simplistic mechanical model (spring model) is used. This model allows to convert local pressure signals into forces. Maybe this is useful for a rigid body (which not even the head is if we include the skin slipping). However, in reality the situation is much more complex in a patient setup, and the relation to 3D motion can be very complex and not locally encoded. The success or failure of the model used in US2020121954A1 could depend upon the immobilization devices, the required equipment that is used and last but not least the invisible elastic moduli of the human body tissues. Some tissues within the same subject may shift extremely in the shoulder region while there is hardly a change to the local pressure signals at all, i.e. e.g. the body is basically doing a shear motion over hips and shoulder with the most relevant pressure signal change e.g. at the head apex or elbows and heels.

In contrast, examples generate a subject position model from repeatedly acquired spatial maps and repeatedly acquired arrays of pressure data. The subject position model is not limited by the simplistic spring model used in US2020121954A1.

In another example generating the subject position model comprises determining the subject position using the spatial map and determining a mapping between the subject position and the array of pressure data. The spatial map and the array of pressure data could for example be acquired repeatedly for several minutes. This would provide a dataset which is descriptive of the typical movements of the subject while the subject is on the subject surface.

In another example, the mapping between the subject position and the array of pressure data is determined by generating a lookup table mapping the array of pressure data to the subject position. For example, the subject position may be divided into bins or the range of motion of the subject in different ways may be divided into bins. The repeatedly acquired spatial map and pressure data may then for example be averaged. This may provide for an accurate and effective means of providing the subject position when the array of pressure data contains noise.

In another example, the mapping between the subject position and the array of pressure data is determined by training a neural network using the determined subject position as ground truth data and the repeatedly acquired array of pressure data as trial data. The neural network after training is configured to output the subject position in response to receiving the array of pressure data as input. The neural network may in some examples be pre-trained using data acquired from a variety of subjects and spatial maps acquired with the pressure data. This may for example provide a means of rapidly training or modifying the neural network to work with a particular subject and position.

In one example, the neural network could be a recurrent neural network.

In another example, the neural network comprises any one of the following: multiple convolutional layers, multiple fully connected layers, and combinations thereof. In some examples the neural network may have more complicated structures. For example, the multiple convolutional layers could be incorporated into a U-Net.

To build a neural network-based system for tracking the position of a subject for use in a medical imaging system such as an MRI or CT system using pressure data from a distributed array of pressure sensors. There are several possible implementations as described above: convolutional neural network (CNN) layers, fully connected layers, or a combination of the two may be used. CNN layers are well-suited for spatially distributed data, as they can capture local patterns in the pressure distribution, potentially learning how the pressure changes correspond to different subject positions. This could be followed by or alternatively replaced by several fully connected layers to aggregate these spatial features and make predictions about the subject's position.

Alternatively, recurrent neural networks (RNNs) could be integrated to account for temporal changes in pressure data over time. RNNs can function as digital filters and may be able to filter noise which is contained in the pressure measurements.

For training the neural network, one may collect a synchronized dataset (temporally registered) comprising of arrays of pressure data from the array of spatially distributed pressure sensors and corresponding ground-truth position data, which could be provided using the remote spatial mapping system. Spatial maps acquired by or derived from the remote spatial mapping system may be time-synchronized (temporally registered) with the arrays of pressure data to ensure that the input-output pairs are aligned. During preprocessing, the pressure data may be transformed into a suitable format, such as a spatial grid, while the spatial maps would provide corresponding labels representing the subject's position, which in some examples could be expressed as 2D or 3D coordinates. The training process would involve feeding batches of arrays of pressure data into the neural network and using a loss function (e.g., mean squared error) to measure the difference between the subject's predicted position and the actual position obtained from the spatial map.

Backpropagation may be applied to update the network's weights, and an optimizer like Adam or SGD could be used to minimize the loss. To improve generalization, techniques such as data augmentation (e.g., adding noise to pressure readings) and regularization methods (like dropout or L2 regularization) could be applied. The model's performance may be validated using a separate validation dataset to monitor overfitting and fine-tune hyperparameters like learning rate, network architecture, and batch size. Once trained, the model could infer the subject's position in real time based on incoming pressure data alone.

In one implementation of the above example would be to use the spatial map to identify which nodes of the spatially distributed pressure sensors are sensitive to body motion and which nodes will mostly contribute only noisy signals. In a further example, the selection of the "patient-motion sensitive" nodes can be made organ dependent: for example, the spatial map could be used to identify the nodes sensitive to head motion, or the nodes sensitive to respiratory motion.

In a further example, the spatial map is used to estimate subject volumes and, from there, the mass that is applied to each pressure sensor. This data can be used to weight / correct the signals received from each pressure sensor. For example, a pressure signal divided by the volume, or the mass, could be derived as input for the subject position.

In another example, determining the subject position comprises fitting a mass model of the subject to the spatial map. The mass model may for example define regions of the subject body and define the individual mass of these regions and how they are allowed to move relative to each other. For example, in order to generate data or better mapping, the mass model of the subject may be adjusted using several steps. First the position of the mass model may be roughly adjusted until it matches the measured spatial map. For example, As was mentioned, the mass model may contain a mass distribution which is typical of a subject as well as have joint movement which models that of a real subject. From examining the spatial map, the spatial mass distribution of the subject may be estimated and then the relative positions of mass model are adjusted until it fits this estimated mass distribution.

As a second step, the relative position of the regions of the body has their position further adjusted by simulating the pressure measurements caused by the mass model reposing of the subject support. The positioning of the mass model may then have its position more finely adjusted until the simulated pressure measurements matches the measured array of pressure data. The fitting of the mass model to the subject may be beneficial because this may be used, in some instances, for generating more training data and also may for example be used for modeling the movement of the subject. For example, if there are pressure measurements that do not fit to acquired mapping data, one could adjust the position of the mass model and see the effect of this on the pressure sensors.

In another example, execution of the machine-executable instructions further causes the computational system to adjust a sensitivity of the array of pressure data using the mass model during generation of the subject position model and during providing the subject position. Adjusting the sensitivity may have several benefits depending upon the example. In some instances, the anatomy of interest may be limited, for example, only the head region. In this case, the sensitivity of some other sensors, which is related to body parts which are distal to the head, may be reduced.

In another example, determining the subject position comprises obtaining anatomical key points by entering the spatial map into an anatomical key point identification neural network. The generation of the subject position model comprises usage of the anatomical key points. Anatomical key points are key points which may indicate the location of particular anatomical locations of the subject. The anatomical key point identification neural networks have been used in a variety of system such as commercially available video game systems]. In these systems an image is input into the anatomical key point identification neural network and the locations of various anatomical positions are marked in the image. This may provide detailed information about the positioning of the subject. A mass model for example may also be fit to the anatomical key points for providing more and better data for training or modeling the movement of the subject.

In another example, the memory further stores an object recognition module configured to identify inanimate objects in the spatial map. For example, there may be such things as coils, immobilization devices / holders, other medical devices or objects which may be on the subject support with the subject. Execution of the machine-executable instructions further causes the computational system to identify inanimate objects by inputting the spatial map into the object recognition module. Execution of the machine-executable instructions further causes the computational system to separate pressure sensor data from the pressure sensors within a predetermined distance of the inanimate objects during generation of the subject position model and during providing of the subject position. For example, the location of some inanimate objects may prevent movement of the subject having much effect on this particular pressure sensor element. Separating the pressure sensor data may for example mean splitting the data related to the object from the subject so that it does not affect the determination of the positions.

In some instances, the trained object recognition module may be a collection of neural networks which are used together. For example, the trained object recognition module may comprise a neural network which is trained to recognize various anatomical key points. This may provide a detailed information on the position of a subject on the subject support. In other cases, one of these neural networks may provide for a convolutional neural network which is able to output bounding boxes which identify the location of various objects or appliances which may also be placed on the subject surface. Both the identification of anatomical key points as well as the identification of the location of objects are possible using currently available neural networks. The combination of the two may provide a flexible means of identifying both subjects and inanimate or animate objects placed on the subject support.

In another example, the remote spatial mapping system is any one of the following: an optical camera, an infra-red camera, an optical remote spatial mapping system, and a three-dimensional camera. The use of various optical types of imaging may for example give detailed information about the position of the subject.

In another example, the remote spatial mapping system is a radar system, a LIDAR system, or an ultrasound system. All these imaging modalities may provide an alternative to using optical methods for determining the position of the subject.

In another example, the spatially distributed array of pressure sensors comprises a spatially distributed array of fiber optic pressure sensors Fiber optic pressure sensors as used herein encompass pressure sensors which operate by the transmission of light through optical fibers and the interaction of light with the pressure sensor. Various types of fiber optic pressures sensors may be used such as intensity-based sensors, interferometer -based sensors, and Fabry-Perot sensors. The advantage of using fiber optic pressure sensors is that they are compatible with several different modes of medical imaging or tomographic medical imaging in particular. For example, the fiber optic pressure sensors are compatible with magnetic resonance imaging systems and with computed tomography systems. The array of pressure data may for example represent the value of pressure measured by an individual fiber optic pressure sensor at a particular time or interval of time.

The combination of the fiber optic pressure sensors and the remote spatial mapping system may be particularly beneficial. The fiber optic pressure sensors are straight forward to implement and MRI compatible. However, they may be noisy, and this noise may differ from fiber optic pressure sensor to fiber optic pressure sensor. In examples, the spatial map is repeatedly acquired as the array of pressure data is repeatedly acquired. As the data for the generation of the subject position model is acquired, the model is also then calibrated in terms of the noise profile of the individual fiber optic pressure sensors.

In another example, the spatially distributed array of pressure sensors comprises a spatially distributed array of fiber optic Bragg grating pressure sensors. This is a particular example of a type of fiber optic pressure sensor which would be accurate and economical to implement.

In another example, the spatially distributed array of pressure sensors comprises a spatially distributed array of pressure-cavity sensors. These pressure-cavity sensors may for example be cavities filled with air connected via tubes to an air-flow sensor.

In another example, the spatially distributed array of pressure sensors comprises a spatially distributed array of capacitive pressure sensors. Capacitive pressure sensors would use wiring; however, they could be made MRI compatible by follow RF-safe design principles e.g. using traps, transformers or highly resistive wires. These techniques are currently used for the implementation of magnetic resonance imaging RF coils. In another example, the medical system further comprises a medical imaging system. Execution of the machine-executable instructions further causes the computational system to control the medical imaging system to acquire medical image data descriptive of the subject. Execution of the machine-executable instructions further causes the computational system to adjust the medical imaging system during the acquisition of the medical image data using the subject position. For example, this may be used to adjust the settings on the medical imaging system. For example, this may be useful in gating the acquisition of the medical image data. In other examples it may be used to control or drive motion correction algorithms.

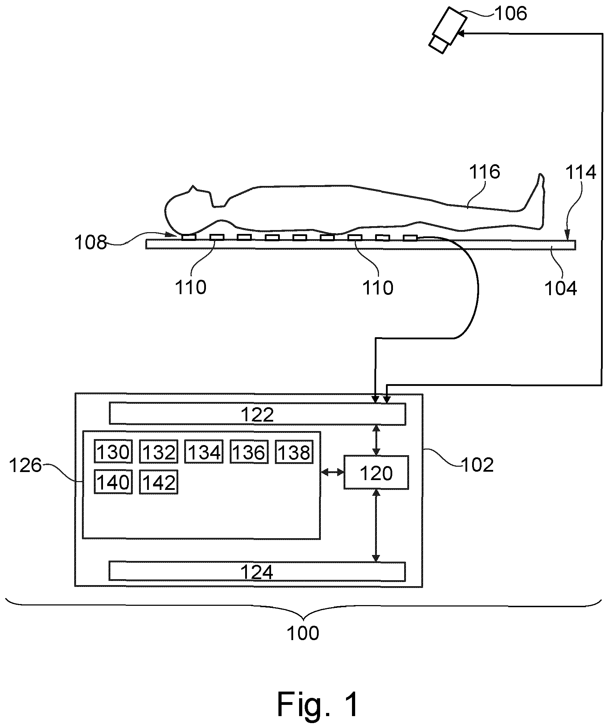

Fig. 1 illustrates an example of a medical system 100. This medical system 100 is shown as comprising a computer 102 that is connected to a subject support 104 and a camera system 106. The camera system 106 functions as a remote spatial mapping system in this example. An array of spatially distributed pressure sensors 108 are distributed on a subject surface 114 of a subject support 112. Several individual pressure sensors 110 are individually marked. A subject 116 is reposing on the subject support 104.

The computer 102 is shown as comprising a computational system 120 that is connected to a hardware interface 122 and an optional user interface 124. The computational system 120 is intended to represent one or more computational systems or computers located at one or more locations. The hardware interface 122 enables the computational system 120 to communicate and control the other components of the medical system 100. The user interface 124, if it is present, may be used to operate and control the medical system 100.

The computational system 120 is also shown as being connected to a memory 126. The memory 126 is intended to represent various types of memories which may be connected or accessible to the computational system 120.

The memory 126 is shown as storing machine-executable instructions 130. The machine-executable instructions enable the computational system 120 to perform such tasks as controlling other components of the medical system 100 as well as performing numerical and image processing tasks. The memory 126 is further shown as containing multiple spatial maps 132 and multiple arrays of pressure data 134. The multiple spatial maps 132 were acquired using the camera system 106 and the multiple arrays of pressure data 134 were acquired using the array of spatially distributed pressure sensors 108. There may be a temporal correlation between the individual spatial maps 132 and the individual arrays of pressure data 134.

The memory 126 is then shown as optionally having a pre-trained neural network 136. The pre-trained neural network 136 may for example have been trained on spatial maps 132 and arrays of pressure data 134 obtained from different subjects 116 in a variety of situations. In this case, the multiple spatial maps 132 and the multiple arrays of pressure data 134 are then used to train the pre-trained neural network 136 into the subject position model 138. In this example, the pre-trained neural network 136 is then adjusted to the particular subject 116 and the particular situation that the subject 116 is on the subject surface 114. In other examples, the pre-trained neural network 136 may not be present and the subject position model 138 is derived entirely from the multiple spatial maps 132 and the multiple arrays of pressure data 134. For example, the subject position model 138 in this case may be a lookup table.

Once the subject position model 138 has been generated or adjusted, the array of spatially distributed pressure sensors 108 may be used to determine a position of the subject 116. The memory 126 is further shown as containing newly acquired array of pressure data 140. This is then input into the subject position model 138 to obtain a subject position 142. The subject position 142 is then able to be determined without the use of the camera system 106.

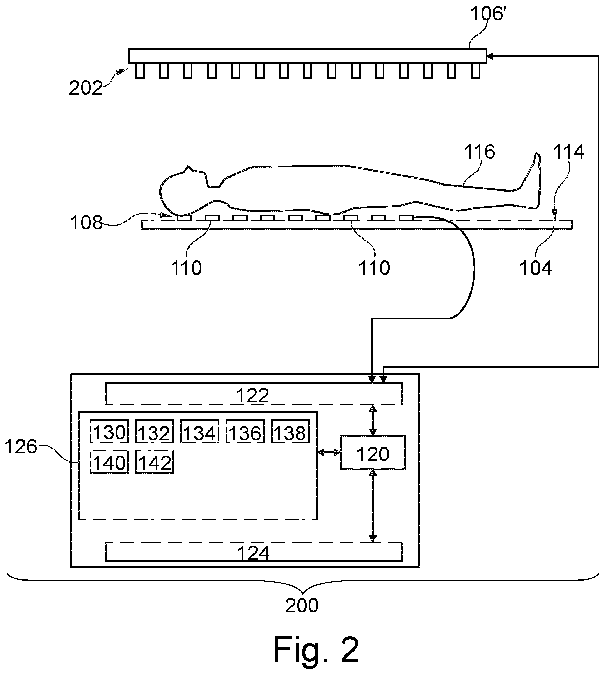

Fig. 2 illustrates a further example of a medical system 200. The medical system 200 illustrated in Fig. 2 is similar to that depicted in Fig. 1 except the camera system 106 has been replaced with a radar array, LIDAR array, or ultrasound array 106'. There are a number of transceiver elements 202 that are positioned in an array above the subject 116. The use of the remote spatial mapping system 106' provides an alternative to using an optical system as is illustrated in Fig. 1.

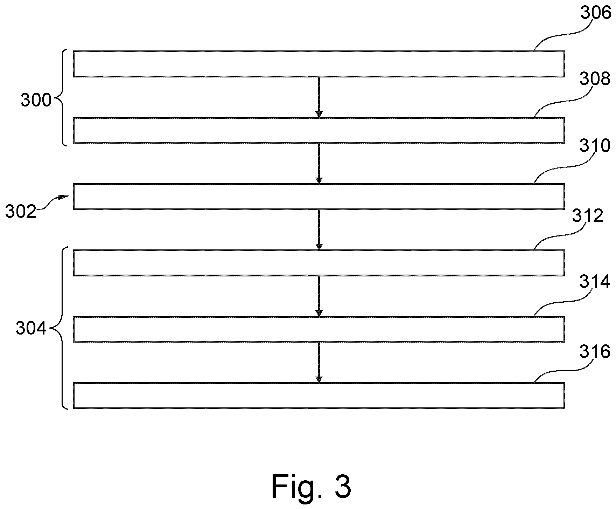

Fig. 3 shows a flowchart which illustrates a method of operating the medical system 100 of Fig. 1 or the medical system 200 of Fig. 2. The method is divided into three phases. There is a calibration portion 300, a generation of the subject position model portion 302, and then motion tracking portion 304 using the subject position model 138.

During the calibration 300 the spatial maps 132 are repeatedly acquired using the remote spatial mapping system which in this example is the camera system 106. In step 308, the array of pressure data 134 is repeatedly acquired. As was mentioned before, the arrays of pressure data 134 and the multiple spatial maps 132 are temporally correlated or labeled as to their time. In the next phase 302, the method comprises generating 310 the subject position model 138 using the repeatedly acquired spatial map 132 and the array of pressure data 134. The subject position model 138 is configured to output a subject position 142 in response to receiving the array of pressure data 140. During the subject motion tracking phase 304, steps 312, 314, and 316 are performed. In step 312 the array of pressure data 140 is repeatedly acquired. After the array of pressure data is acquired 140, step 314 is performed. In step 314 the subject position 142 is received in response to inputting the array of pressure data 140 into the subject position model 138. In step 316 the subject position 142 is then provided. For example, the subject position 142 may be provided to an operator on the user interface 124 and it may also be incorporated into the control of a medical imaging system such as a computed tomography or magnetic resonance imaging system.

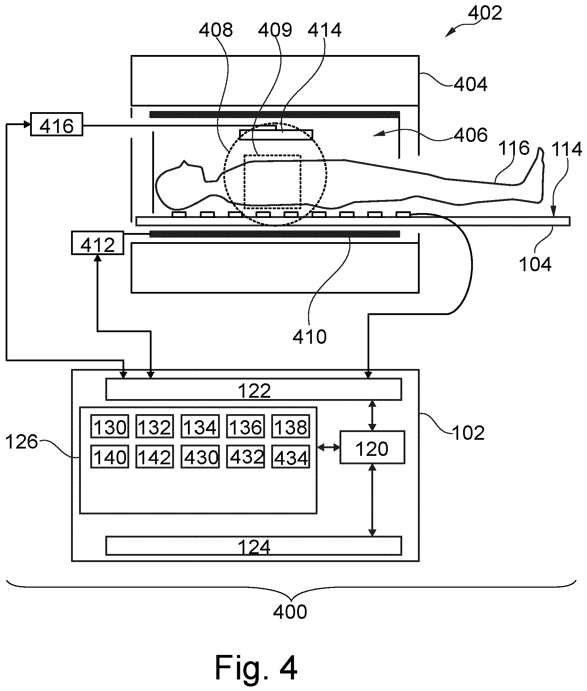

Fig. 4 illustrates a further example of a medical system 400. In this example the medical system 400 comprises the magnetic resonance imaging system 402 along with the subject support 104. The camera system 106 as illustrated in Fig. 1 or the radar array or ultrasound array 106' as illustrated in Fig. 2 could be incorporated outside of the magnetic resonance imaging system 402 or at a different location. For example, in the first case, the remote spatial mapping system 106, 106' could be immediately outside the bore of the magnetic resonance imaging system 402 and then the subject support 104 along with the subject 106 is injected into the bore 406. In other examples the subject support 104 may be removable from the magnetic resonance imaging system 402 and the camera system 106 or the radar array or ultrasound array 106' could be at a different location. The features of Figs. 1 and/or 2 may be combined with the features of Fig. 4.

The magnetic resonance imaging system 402 comprises a magnet 404. The magnet 404 is a superconducting cylindrical type magnet with a bore 406 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. A field of view 309 is shown within the imaging zone 308. The k-space data are acquired for the field of view 409. The region of interest could be identical with the field of view 409 or it could be a sub volume of the field of view 409. A subject 116 is shown as being supported by the subject support 104 such that at least a portion of the subject 116 is within the imaging zone 408 and the field of view 409.

Within the bore 406 of the magnet there is also a set of magnetic field gradient coils 410 which is used for the acquisition of measured k-space data to spatially encode magnetic spins within the imaging zone 408 of the magnet 404. The magnetic field gradient coils 410 are connected to a magnetic field gradient coil power supply 412. The magnetic field gradient coils 410 are intended to be representative. Typically, magnetic field gradient coils 410 contain three separate sets of coils for spatial encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio frequency coil 414 is connected to a radio frequency transceiver 416. The radio frequency coil 414 and radio frequency transceiver 416 may be replaced by separate transmit and receive coils and a separate transmitter and receiver. It is understood that the radio frequency coil 414 and the radio frequency transceiver 416 are representative. The radio frequency coil 414 is intended to also represent a dedicated transmit antenna and a dedicated receive antenna. Likewise, the transceiver 416 may also represent a separate transmitter and receiver. The radio frequency coil 414 may also have multiple receive/transmit elements and the radio frequency transceiver 416 may have multiple receive/transmit channels. The transceiver 416 and the gradient controller 412 are shown as being connected to the hardware interface 122 of the computer system 102.

The magnetic resonance imaging system 402 is shown as being controlled by the computer system 402. The memory 126 is further shown as containing pulse sequence commands 430. The pulse sequence commands are commands or data which may be converted into commands to control the magnetic resonance imaging system 402 for a particular magnetic resonance imaging protocol. The memory 126 is further shown as containing k-space data 432 that was acquired by controlling the magnetic resonance imaging system 402 with the pulse sequence commands 430. The memory 126 is also shown as containing an optional magnetic resonance image 434 that was reconstructed from the k-space data 432.

The subject motion signal 142 may be used in different ways when controlling the magnetic resonance imaging system with the pulse sequence commands 430. For example, the subject motion signal 142 could be used to trigger or engage the acquisition of the k-space data 432. In other instances it may be used for example to cancel or restart the acquisition of the k-space data 432 if it is determined that the subject 116 has moved too much.

Motion correction can also be performed on the k-space data 432. For example there may be k-space navigators included in the k-space data which enables self-navigation or a technique for self-motion correction such as PROPELLER may be used. The motion estimated with the k-space data may also be combined with the subject position data to obtain a better estimate of the subject motion during the reconstruction of a magnetic resonance image from the k-space data 432.

It should also be noted that the use of a magnetic resonance imaging system 402 in Fig. 4 and the following Figs. is only an example. The magnetic resonance imaging system 402 may for example also be replaced with a computed tomography system.

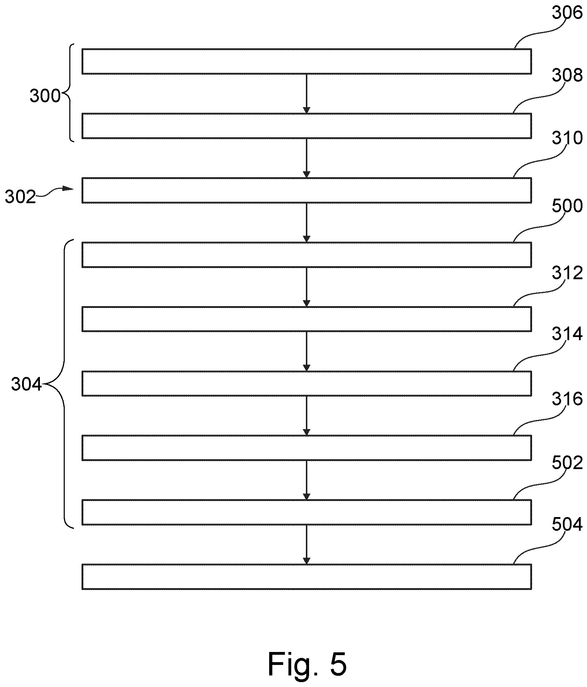

Fig. 5 shows a flowchart which illustrates a method of operating the medical system 400 of Fig. 4. The method starts with steps 306, 308, and 310 as was illustrated in Fig. 3. In step 500, the medical imaging system 402 is controlled to acquire the medical image data 432. In this case the medical imaging system is a magnetic resonance imaging system 402 and the medical image data is the k-space data 432. In the case of a computed tomography system the medical image data would be X-ray profiles. Steps 312, 314, and 316 are then performed, as was illustrated in Fig. 3. In step 502 the medical imaging system 402 is adjusted during the acquisition of the medical image data 432 using the subject position. Step 504 is optional. In step 504 the medical image 434 is reconstructed from the medical image data 432. In some instances, the medical image data 432 may be sent to a remote or cloud-based server to perform the image reconstruction.

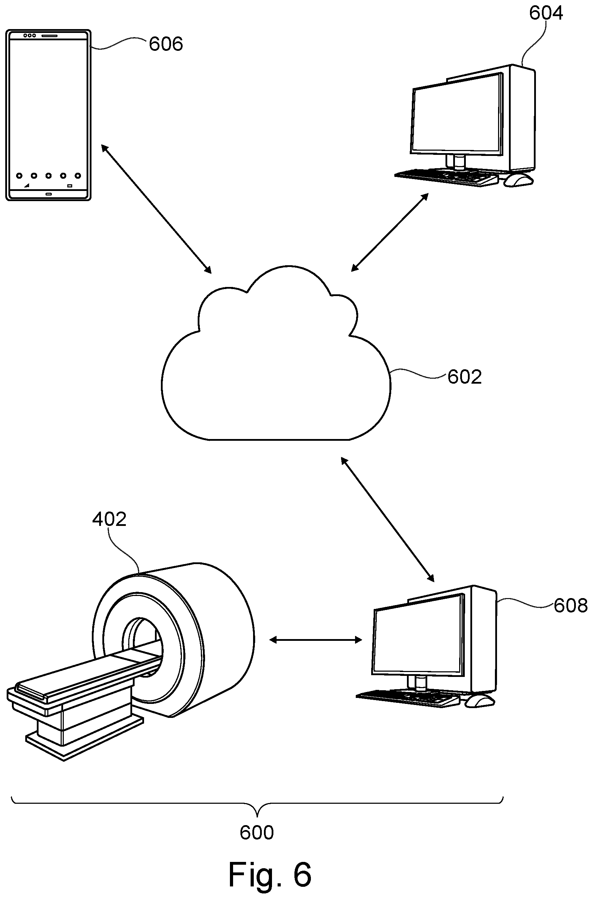

Fig. 6 illustrates a further example of a medical system 600. The medical system 600 comprises the medical imaging system (i.e., the magnetic resonance imaging system 402 of Fig. 4) as well as a cloud-based computing system 602, a radiology workstation 604, a handheld telecommunications device 606, and a local controller 608. The functionality of the computer 102 depicted in Figs. 1, 2, and 4 can be distributed amongst the various devices 602, 604, 606, and 608. The cloud-based computing system 602 could be made up of networked servers and/or virtual machines available over the internet or other network.

A typical configuration would be to use the local controller 608 to control the medical imaging system 402 to acquire the medical image data (i.e., k-space data) 432. The reconstruction of the medical image may be performed by the cloud-based computing system 602. The resulting medical image could then be made available to the workstation 604, the handheld telecommunications device 606, and the local controller 608.

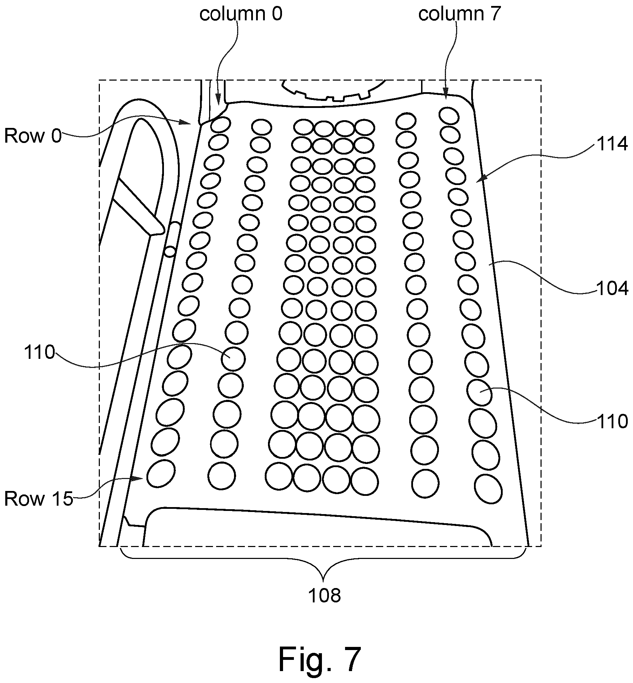

Fig. 7 illustrates an example of a subject support 104 that has an array of spatially distributed pressure sensors 108 implemented using fiber optic pressure sensors on the subject surface 114. In this example the arrays have 8 columns and 16 rows and is sufficient to provide sensors for the upper torso of a subject. The distribution of sensors 110 is sufficient for monitoring such things as breathing, heart signal, and also fine body movement such as finger movement.

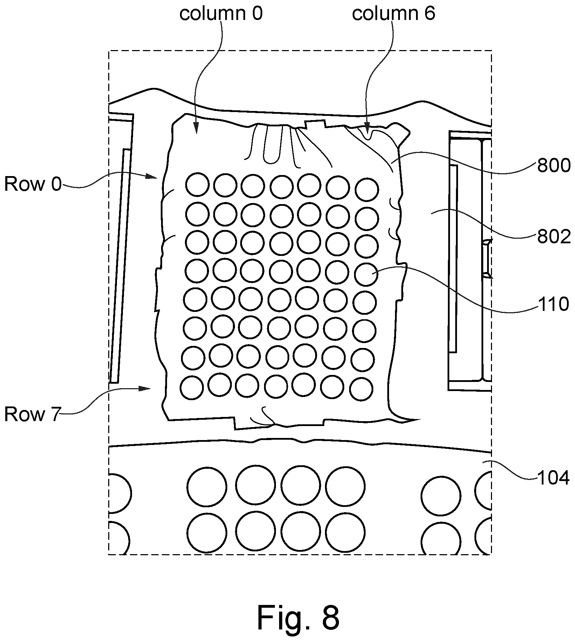

Fig. 8 shows a further view of the subject support 104 depicted in Fig. 7. There is additionally a view of a head support 802 with additional sensors 800 that are added to the array. In this example the sensors are distributed to measure the torso in Fig. 7 and additionally there are sensors to measure the movement of the subject's head. As is illustrated in Figs. 7 and 8, it is straightforward to modify existing subject supports and be able to monitor either the torso or extremities of the subject effectively.

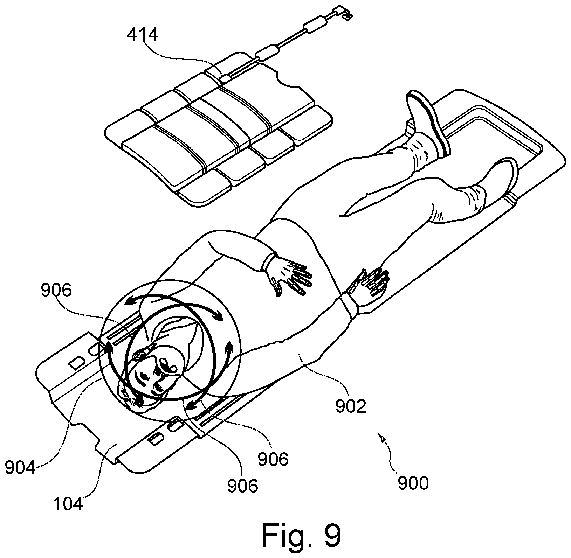

Fig. 9 illustrates a simulation model 900. A mass model 902 of the subject 116 is shown as reposing on the subject support 104. A magnetic resonance imaging coil 414 is shown in an exploded view. In this example the motion of the subject's head is modeled. The circle 904 indicates the head region of the mass model 902. The lines labeled 906 indicate possible degrees of freedom of movement for the head. The head region 904 can be articulated around the possible movements 906 to generate synthetic data which may be used to train the subject-to-position model 138.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: subject support
- 106: camera system (remote spatial mapping system)
- 106': radar array, LIDAR array, or ultrasound array
- 108: array of spatially distributed pressure sensors
- 110: individual pressure sensor
- 112: subject support
- 114: subject surface
- 116: subject
- 120: computational system
- 122: hardware interface
- 124: user interface
- 126: memory
- 130: machine executable instructions
- 132: multiple spatial maps
- 134: multiple arrays of pressure data
- 136: pretrained neural network
- 138: subject position model
- 140: newly acquire array of pressure data
- 142: subject position
- 200: medical system
- 300: calibration portion
- 302: generation of subject position module portion
- 304: motion tracking portion
- 306: repeatedly acquire the spatial map from the remote spatial mapping system
- 308: repeatedly acquire the array of pressure data
- 310: generate a subject position model using the repeatedly acquired spatial map and the array of pressure data
- 312: repeatedly acquire the array of pressure data from the array of spatially distributed pressure sensors
- 314: repeatedly receive the subject position by inputting the array of pressure data into the subject position model
- 316: repeatedly provide the subject position
- 400: medical system
- 402: magnetic resonance imaging system
- 404: magnet
- 406: bore of magnet
- 408: imaging zone
- 409: field of view
- 410: magnetic field gradient coils
- 412: magnetic field gradient coil power supply
- 414: radio frequency coil
- 416: transceiver
- 430: pulse sequence commands
- 432: k-space data (medical image data)
- 434: magnetic resonance image
- 500: controlling the medical imaging system to acquire medical image data descriptive of the subject
- 502: adjust the medical imaging system during the acquisition of medical image data using the subject position
- 800: additional sensors for array
- 802: head support
- 900: simulation model
- 902: mass model of subj ect
- 904: head region
- 906: possible movements

## Claims

1. A medical system (100, 200, 400, 600) comprising:
- a subject support (104) comprising a subject surface (114) configured for receiving a subject (116), wherein the subject surface comprises an array of spatially distributed pressure sensors (108) configured for optically measuring an array of pressure data (134, 140);
- a remote spatial mapping system (106, 106') configured for measuring a spatial map (132) descriptive of objects on the subject surface, wherein the objects on the subject surface comprise the subject, wherein the remote spatial mapping system is configured to register the spatial map to the array of spatially distributed pressure sensors;
- a memory (126) storing machine executable instructions (130);
- a computational system (120), wherein execution of the machine executable instructions causes the computational system to:
- perform a calibration (300) comprising:
- repeatedly (306) acquire the spatial map from the remote spatial mapping system;
- repeatedly (308) acquire the array of pressure data, wherein the array of pressure data is temporally correlated to the spatial map;
- generate (302, 310) a subject position model (138) using the repeatedly acquired spatial map and the array of pressure data, wherein the subject position model is configured to output a subject position (142) in response to receiving the array of pressure data;
- perform subject motion tracking (304) comprising:
- repeatedly (312) acquire the array of pressure data (140) from the array of spatially distributed pressure sensors;
- repeatedly (314) receive the subject position by inputting the array of pressure data into the subject position model; and
- repeatedly (316) provide the subject position.

2. The medical system of claim 1, wherein generating the subject position model comprises determining the subject position using the spatial map and determining a mapping between the subject position and the array of pressure data.

3. The medical system of claim 2, wherein the mapping between the subject position and the array of pressure data is determined by generating a lookup table mapping the array of pressure data to the subject position.

4. The medical system of claim 2, wherein the mapping between the subject position and the array of pressure data is determined by training a neural network (136) using the determined subject position as ground truth data and the repeatedly acquired array of pressure data as trial data, and wherein the neural network after training is configured to output the subject position in response to receiving the array of pressure data as input.

5. The medical system of claim 4, wherein the neural network comprises a recurrent neural network and/or comprises any one of the following: multiple convolutional layers, multiple fully connected layers, and combinations thereof.

6. The medical system of any one of claims 2 through 5, wherein determining the subject position comprises fitting a mass model (902) of the subject to the spatial map.

7. The medical system of claim 6, wherein execution of the machine executable instructions further causes the computational system to adjust a sensitivity of the array of pressure data using the mass model during generation of the subject position module and during providing of the subject position.

8. The medical system of any one of claims 2 through 7, wherein determining the subject position comprises obtaining anatomical key points by inputting the spatial map into an anatomical key point identifying neural network, wherein the generation of the subject position model comprises usage of the anatomical key points.

9. The medical system of any one of the preceding claims, wherein the memory further stores an object recognition module configured to identify inanimate objects in the spatial map, wherein execution of the machine executable instructions further causes the computational system to:
- identify inanimate objects by inputting the spatial map into the object recognition module; and
- separate pressure sensor data from pressure sensors within a predetermined distance of the inanimate objects during generation of the subject position module and during providing of the subject position.

10. The medical system of any one of the preceding claims, wherein the remote spatial mapping system is any one of the following: an optical camera, an infrared camera, an optical remote spatial mapping system, a three-dimensional camera, a radar system, a LIDAR system, and an ultrasound system.

11. The medical system of any one of the preceding claims, wherein the spatially distributed array of pressure sensors is any one of the following: a spatially distributed array of fiber optic pressure sensors, a spatially distributed array of pressure-cavity sensors, a spatially distributed array of capacitive pressure sensors, and a spatially distributed array of fiber optic Bragg grating pressure sensors.

12. The medical system of any one of the preceding claims, wherein the medical system further comprises a medical imaging system (402), wherein execution of the machine executable instructions further causes the computational system to:
- controlling (500) the medical imaging system to acquire medical image data (432) descriptive of the subject; and
- adjust (502) the medical imaging system during the acquisition of medical image data using the subject position.

13. The medical system of claim 12, wherein the medical system is a magnetic resonance imaging system (402) or a computed tomography system.

14. A method of operating a medical system (100, 200, 400, 600), wherein the medical system comprises a subject support (104) comprising a subject surface (114) configured for receiving a subject, wherein the subject surface comprises an array of spatially distributed pressure sensors (108) configured for optically measuring an array of pressure data, wherein the medical system further comprises a remote spatial mapping system (106, 106') configured for measuring a spatial map (132) descriptive of objects on the subject surface, wherein the remote spatial mapping system is configured to register the spatial map to the array of spatially distributed pressure sensors, wherein the method comprises:
- performing a calibration (300) comprising:
- repeatedly (306) acquiring the spatial map from the remote spatial mapping system;
- repeatedly (308) acquiring the array of pressure data, wherein the array of pressure data is temporally correlated to the spatial map;
- generating (302, 310) a subject position model (138) using the repeatedly acquired spatial map and the array of pressure data, wherein the subject position model is configured to output a subject position in response to receiving the array of pressure data;
- performing subject motion tracking (304) comprising:
- repeatedly (306) acquiring the array of pressure data (140) from the array of spatially distributed pressure sensors;
- repeatedly (308) receiving the subject position by inputting the array of pressure data into the subject position model: and
- repeatedly (310) providing the subject position.

15. A computer program comprising machine executable instructions (130), wherein execution of the machine executable instructions causes a computational system to perform a method according to claim 14.
